# EUROPEAN PATENT APPLICATION

(11) **EP 3 476 279 A2**
(43) Date of publication of application: **01.05.2019**
(21) Application number: 18200673.4
(22) Date of filing: 16.10.2018
(51) Int. Cl.: A61B 5/00, A61F 2/38, A61F 2/48, A61B 90/00, G06T 7/00, A61B 5/107, A61B 6/03

(54) **ARTIFICIAL JOINT WITH ABRASION MEASURING ELEMENT AND METHOD FOR MEASURING ABRASION OF ARTIFICIAL JOINT**

(30) Priority: 24.10.2017 KR 20170138616
(71) Applicant: Paik, Hae Sun, Seoul 03075 (KR)
(72) Inventor: Paik, Hae Sun, Seoul 03075 (KR)
(74) Representative: Sanger, Phillip Simon

(57) **Abstract**

Provided are an artificial joint with an abrasion measuring element and a method for measuring abrasion of an artificial joint, in which the abrasion of the artificial joint is determined by any one selected from a sensor device, a marker, and an information pattern.

## Description

### TECHNICAL FIELD

The present invention relates to an artificial joint with an abrasion measuring element capable of measuring an abrasion amount of an artificial knee joint and a method for measuring abrasion of an artificial joint.

### BACKGROUND ART

In general, an artificial knee joint is constituted by a femoral component, a tibial component positioned below the femoral component, and a tibial plate positioned between the femoral component and the tibial component, which is an artificial cartilage (*see* Korean Patent Registration No. 10-1769125 "Artificial knee joint comprising surface-contact protrusions"). The femoral component and the tibial component are made of metals such as cobalt and a chromium alloy. The tibial plate is made of plastic such as polyethylene and polyester. The lifespan of an artificial knee joint is known to be about 15 years. The abrasion of the tibial plate made of plastic determines the lifespan of the artificial knee joint. When a tibial plate having a cartilage function is worn out and pain occurs again, it should be replaced with a new artificial knee joint by artificial knee joint replacement arthroplasty. The abrasion of the tibial plate may be confirmed by X-ray or computed tomography (CT) imaging. However, in the X-ray or CT, imaging may be cumbersome and may be a burden on patients in cost. In addition, X-ray and CT are the most commonly used imaging devices, but there is a risk of repetitive radiation exposure caused by imaging.

Therefore, it is urgently required to develop a device capable of easily checking the abrasion amount of an artificial joint.

### SUMMARY OF THE INVENTION

The present invention has been made in an effort to provide an artificial joint with an abrasion measuring element and a method for measuring abrasion of an artificial joint capable of determining abrasion of the artificial joint through any one selected from a sensor device, a marker, and an information pattern provided on the artificial joint.

An exemplary embodiment of the present invention provides an artificial joint with an abrasion measuring element, in which the abrasion of the artificial joint may be determined by any one selected from a sensor device, a marker, and an information pattern provided in the artificial joint.

The sensor devices may be provided on a tibial component and a tibial plate configuring the artificial joint.

The sensor device may include a first sensor provided in the femoral component; and a second sensor provided in the tibial component, in which the abrasion of the artificial joint may be determined by distance information between the first sensor and the second sensor acquired from the first sensor and the second sensor.

The first sensor may be positioned on one side of the femoral component so as not to interfere with the operation of the artificial joint and the second sensor may be positioned directly below the first sensor correspondingly with the first sensor and positioned on one side of the tibial component so as not to interfere with the operation of the artificial joint.

The first sensor and the second sensor may be non-power sensors.

The sensor device may be an abrasion amount measuring sensor, and the abrasion amount measuring sensor may be positioned on one side of the femoral component or the tibial component so as not to interfere with the operation of the artificial joint to measure an amount of abrasive particles of the tibial plate in a body fluid filled in a glenoid cavity.

The abrasion amount measuring sensor may be a non-power sensor.

The first sensor may be attached to a lowest point of the femoral component.

The marker may be provided at either the femoral component or the tibial component configuring the artificial joint, or at both the femoral component and the tibial plate.

A plurality of markers or three or more markers may be provided.

First artificial joint 3D data may be overlapped with imaging 2D data of the artificial joint operated to a patient to determine the abrasion of the artificial joint through abrasion area data where the artificial joint of the 2D data is not overlapped with the artificial joint of the 3D data.

Artificial joint makers of both data may coincide with each other when the 3D data is overlapped with the 2D data.

The 2D data may be X-ray or CT imaging data.

Any one of the information patterns may be provided in a friction region of the artificial joint and the remaining plurality of information patterns may be provided in a region where the friction of the artificial joint does not occur, and first artificial joint 3D data is overlapped with imaging 2D data of the artificial joint operated to a patient to determine the abrasion of the artificial joint through data obtained by comparing the information pattern provided in the friction region of the artificial joint of the 2D data with the information pattern provided in the friction region of the artificial joint of the 3D data.

Artificial joint information patterns of both data may coincide with each other when the 3D data is overlapped with the 2D data.

Product information of the artificial joint may be stored in the information pattern.

Another exemplary embodiment of the present invention provides a method for measuring an artificial joint, the method including: acquiring first artificial joint 3D data; acquiring imaging 2D data of the artificial joint operated to a patient; overlapping the 3D data with the 2D data; and determining abrasion of the artificial joint through an abrasion area of the artificial joint not overlapped with each other when the 3D data is overlapped with the 2D data or determining abrasion of the artificial joint through data obtained by comparing the information pattern provided in the friction region of the artificial joint of the 2D data with the information pattern provided in the friction region of the artificial joint of the 3D data when the 3D data is overlapped with the 2D data.

Artificial joint markers or pattern information of both data may coincide with each other when the 3D data is overlapped with the 2D data.

According to the exemplary embodiment of the present invention, in the artificial joint with the abrasion measuring element and the method for measuring abrasion of the artificial joint, it is possible to determine whether to replace an artificial joint by measuring an abrasion amount of the artificial joint by a sensor device attached to the artificial joint.

Further, it is possible to reduce inconvenience and a burden in cost of X-ray or CT imaging.

Further, it is possible to deviate from a risk of radiation exposure due to X-ray and CT imaging.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

FIG. 1 is an exploded perspective view of an artificial joint with a sensor device according to a first exemplary embodiment of the present invention.
FIG. 2 is a view of measuring an abrasion amount of the artificial joint by the sensor device according to the first exemplary embodiment of the present invention.
FIG. 3 is a use state view of an artificial joint with an abrasion measuring device according to a second exemplary embodiment of the present invention.
FIG. 4 is an installation view of an artificial joint with a marker according to a third exemplary embodiment of the present invention.
FIG. 5 is a view illustrating a process of measuring abrasion of the artificial joint by the marker according to the third exemplary embodiment of the present invention.
FIG. 6 is an installation view of an artificial joint with an information pattern according to a fourth exemplary embodiment of the present invention.

It should be understood that the appended drawings are not necessarily to scale, presenting a somewhat simplified representation of various features illustrative of the basic principles of the invention. The specific design features of the present invention as disclosed herein, including, for example, specific dimensions, orientations, locations, and shapes will be determined in part by the particular intended application and use environment.

In the figures, reference numbers refer to the same or equivalent parts of the present invention throughout the several figures of the drawing.

### DETAILED DESCRIPTION

Hereinafter, preferred exemplary embodiments of the present invention will be described in detail with reference to the accompanying drawings. When reference numerals refer to components of each drawing, it is to be noted that although the same components are illustrated in different drawings, the same components are denoted by the same reference numerals as possible. Hereinafter, a preferred exemplary embodiment of the present invention will be described, but the technical spirit of the present invention is not limited or restricted thereto and the present invention may be modified and variously implemented by those skilled in the art.

In an artificial joint with an abrasion measuring element and a method for measuring abrasion of the artificial joint according to an exemplary embodiment of the present invention, the artificial joint with the abrasion measuring element may determine whether to replace an artificial joint according to the abrasion of the artificial joint using a sensor device, a marker, and an information pattern provided on the artificial joint.

First, a first exemplary embodiment of the present invention will be described.

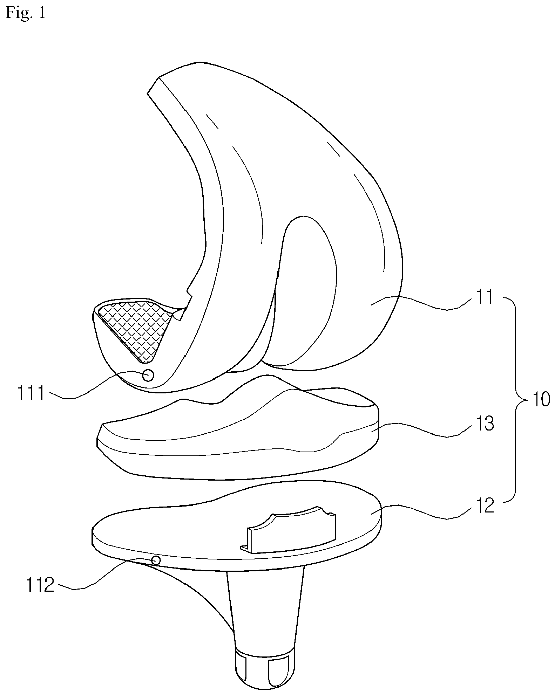

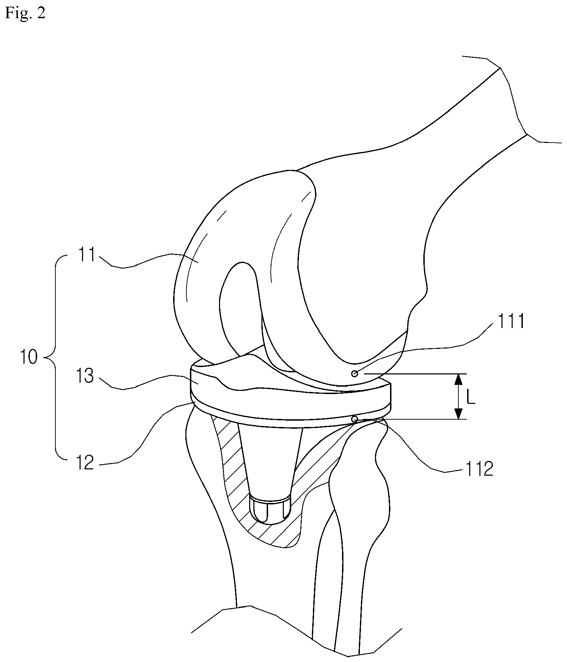

As illustrated in FIGS. 1 and 2, the first exemplary embodiment of the present invention includes an artificial joint 10 and a sensor device provided on the artificial joint 10.

Like an existing artificial joint, the artificial joint 10 includes a femoral component 11, a tibial component 12 positioned below the femoral component 11, and a tibial plate 13 positioned between the femoral component 11 and the tibial component 12 to serve as a cartilage.

The femoral component 11 and the tibial component 12 are made of metals such as cobalt and a chromium alloy. The tibial plate 13 is made of a plastic material such as polyethylene and polyester. The tibial plate 13 is abraded by the friction between the femoral component 11 and the tibial component 12.

The sensor device includes a first sensor 111 and a second sensor 112. The first sensor 111 is provided on the femoral component 11. The second sensor 112 is provided on the tibial component 12. An abrasion amount of the tibial plate 13 may be checked by information on a distance L between the first sensor 111 and the second sensor 112 acquired from the first sensor 111 and the second sensor 112. For example, when the information on the distance L between the first sensor 111 and the second sensor 112 is equal to or smaller than a predetermined value, it is determined that the lifespan of the tibial plate 13 is exhausted. When it is determined that the lifespan of the artificial joint 10 is exhausted due to the abrasion of the tibial plate 13, the exhausted artificial joint is replaced with a new artificial joint through artificial joint replacement arthroplasty.

The first sensor 111 is positioned on a lower side of the femoral component 11 so as not to interfere with the operation of the artificial joint 10. The second sensor 112 is positioned directly below the first sensor 111 correspondingly with the first sensor 111. The second sensor 112 is positioned on an upper side of the tibial component 12 so as not to interfere with the operation of the artificial joint 10. The first sensor 111 and the second sensor 112 are positioned on the same vertical line. The first sensor 111 and the second sensor 112 may be non-power sensors. The first sensor 111 and the second sensor 112 may be wireless sensors.

For example, the first sensor 111 and the second sensor 112 may be designed as wire sensors having a power source, but in this case, unnecessary power may be supplied to the first sensor 111 and the second sensor 112 regardless of a desired operating time. In addition, when the first sensor 111 and the second sensor 112 are the wire sensors, there may be various inconveniences such as exposing wires connected to the first sensor 111 and the second sensor 112 to the outside of the skin. Therefore, the sensors are designed as a non-power wireless sensor to prevent inconvenience after skin sealing.

Core Chips Co., Ltd. (http://nano_korea.blog.me/80137472507) is known as a developer of non-power sensor, and the non-power sensor technology is a well-known technology and the detailed description thereof is omitted.

For example, the first sensor 111 is attached to the lowest point of the femoral component 11 and measures a distance between two points of the first sensor 111 and the second sensor 112. In the artificial joint 10, most of the tibial plate 13 at the rear portion between the femoral component 11 and the tibial component 12 is abraded.

Next, a second exemplary embodiment will be described.

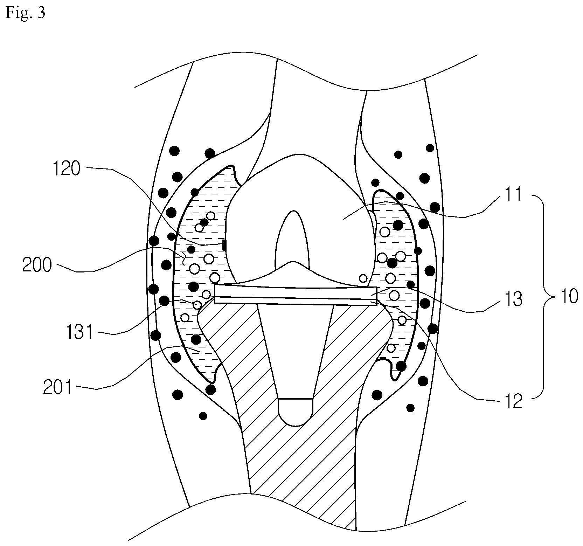

As illustrated in FIG. 3, the sensor device is an abrasion amount measuring sensor 120. The abrasion amount measuring sensor 120 may be a non-power sensor. The abrasion amount measuring sensor 120 may be a wireless sensor.

The abrasion amount measuring sensor 120 is positioned on one side of the femoral component 11 or the tibial component 12 so as not to interfere with the operation of the artificial joint 10. The abrasion amount measuring sensor 120 measures an amount of abrasive particles 131 of the tibial plate 13 in a glenoid cavity 200.

While the tibial plate 13 is abraded by the friction between the femoral component 11 and the tibial component 12, the abrasive particles 131 are separated. The separated abrasive particles 131 are positioned in a body fluid 201 filled in the glenoid cavity 200. The abrasion amount measuring sensor 120 measures an amount of abrasive particles 131 of the tibial plate 13 in the body fluid 201 filled in the glenoid cavity 200. A replacement time of the artificial joint 10 may be determined by measuring the amount of the abrasive particles 131.

For example, when the amount of the abrasive particles 131 is equal to or larger than a set value, it is determined that the lifespan of the tibial plate 13 is exhausted. When it is determined that the lifespan of the artificial joint 10 is exhausted due to the abrasion of the tibial plate 13, the exhausted artificial joint is replaced with a new artificial joint through artificial joint replacement arthroplasty.

As described above, in first and second exemplary embodiments of the present invention, the replacement of the artificial joint may be determined by measuring the abrasion amount of the artificial joint through the sensor device attached to the artificial joint. Further, it is possible to reduce inconvenience and a burden in cost of X-ray or CT imaging. Further, it is possible to deviate from a risk of radiation exposure due to X-ray and CT imaging.

Next, a third exemplary embodiment of the present invention will be described.

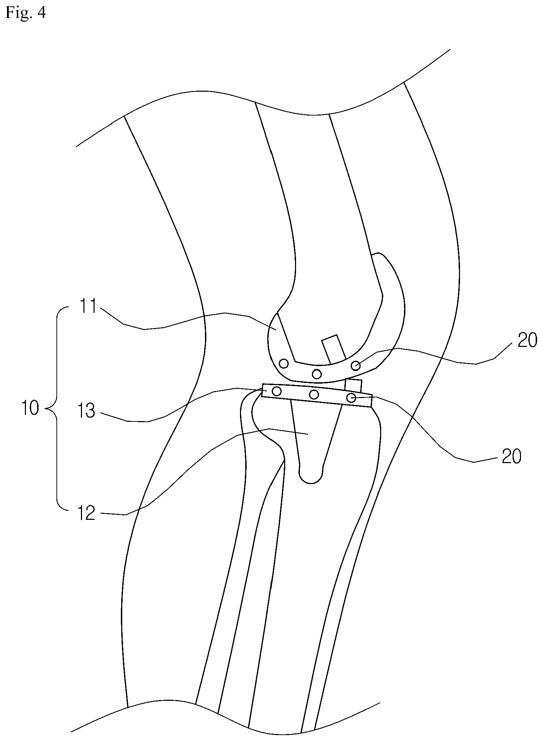

As illustrated in FIG. 4, in the third exemplary embodiment of the present invention, it is possible to determine a replacement time of the artificial joint 10 by measuring the abrasion of the artificial joint 10 using a marker 20 provided on the artificial joint 10.

The marker 20 may be provided at either the femoral component 11 or the tibial plate 13 configuring the artificial joint 10. As another example, the marker 20 may be provided at both the femoral component 11 and the tibial plate 13.

The marker 20 becomes a reference point for accurately overlapping 3D data 103 of the artificial joint with 2D data 102. A plurality of markers 20 or three or more markers may be provided. The 2D data 102 may be imaging data of the artificial joint such as X-ray or CT.

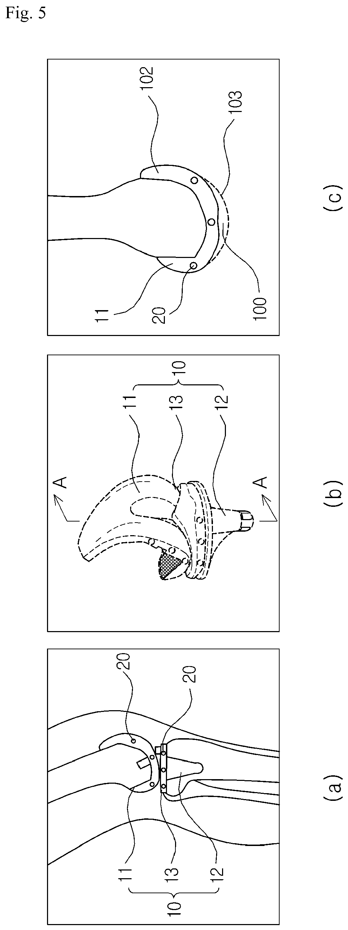

As illustrated in FIG. 5, it is possible to measure the abrasion of the artificial joint using the marker and determine the replacement time of the artificial joint through the abrasion measurement information.

Particularly, the 3D data 103 of the first artificial joint 10 is acquired before operating the artificial joint 10 to the patient (*see* FIG. 5B). The 2D data 102 of the artificial joint 10 for diagnosing the abrasion of the artificial joint 10 is acquired through X-ray or CT imaging of the knee artificial joint 10 of the patient (*see* FIG. 5A).

The acquired 3D data 103 is overlapped with the 2D data 102 (*see* FIG. 5C). Here, since the stereoscopic 3D data 103 freely rotates in all directions, the 3D data 103 is necessarily overlapped based on the 2D data 102.

When the 3D data 103 is overlapped with the 2D data 102, the markers 20 of both data are set as the reference points of the artificial joint 10, and when the markers 20 of both data are overlapped with each other to correspond to each other, the 3D data 103 may be precisely and accurately overlapped with the 2D data 102.

It is possible to determine whether to replace the artificial joint 10 by measuring the abrasion amount of the artificial joint 10 through information on an abrasion area 100 of the artificial joint which does not overlap each other when the 3D data 103 is overlapped with the 2D data 102.

The image of the artificial joint 10 of the 2D data 102 at the time of overlapping is inevitably smaller than the image of the artificial joint 10 of the 3D data 103 because the friction portion is abraded. The abrasion amount of the artificial joint may be measured due to a difference in abrasion area of the artificial joint.

Next, a fourth exemplary embodiment of the present invention will be described.

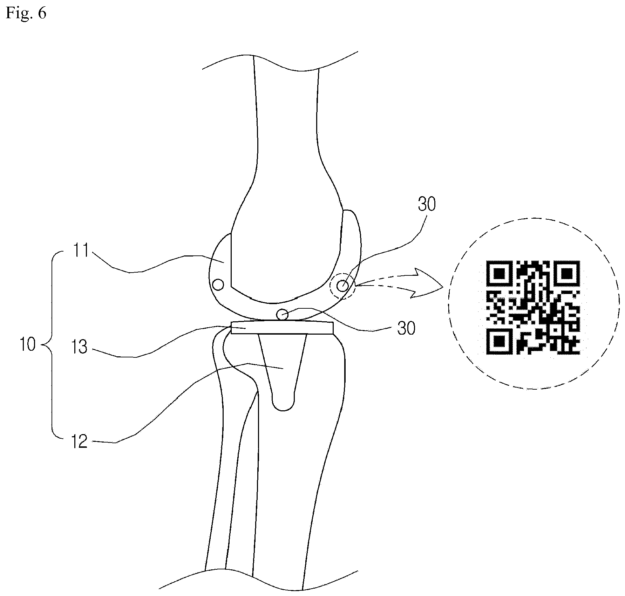

As illustrated in FIG. 6, in the fourth exemplary embodiment of the present invention, it is possible to determine a replacement time of the artificial joint 10 by measuring the abrasion of the artificial joint 10 using an information pattern 30 provided on the artificial joint 10.

The information pattern 30 may be provided at either the femoral component 11 or the tibial plate 13 configuring the artificial joint 10. As another example, the information pattern 30 may be provided at both the femoral component 11 and the tibial plate 13.

At least one information pattern 30 is provided in a region where the artificial joint 10 is abraded. The region where the artificial joint 10 is abraded means a contact region between the femoral component 11 and the tibial plate 13. The remaining plurality of information patterns 30 is provided in a region where the artificial joint 10 is not abraded. The region where the artificial joint 10 is not abraded means a region where the contact between the femoral component 11 and the tibial plate 13 does not occur.

In the information pattern 30, various information such as product information of the artificial joint 10 may be stored. For example, the information pattern 30 may have a specific pattern in which information such as a barcode and a QR code is stored. When the information pattern 30 is scanned, various types of information may be received.

The information pattern 30 becomes a reference point for accurately overlapping 3D data 103 of the artificial joint with 2D data 102. At least three information patterns 30 need to be designed.

The measuring of the abrasion of the artificial joint in the fourth exemplary embodiment may be performed like the third exemplary embodiment. Particularly, the 3D data 103 of the first artificial joint 10 is acquired. The imaging 2D data 102 of the artificial joint 10 is acquired by imaging the patient's knee.

The 3D data 103 is overlapped with the acquired 2D data 102. When the 3D data 103 is overlapped with the 2D data 102, the abrasion of the artificial joint 10 may be determined by comparing the information pattern 30 provided in the abrasion area of the artificial joint 10 of the 3D data 103 with the information pattern 30 provided in the abrasion area of the artificial joint 10 of the 2D data 102. Since the size of the information pattern 30 provided in the abrasion area of the 2D data 102 is inevitably smaller than the size of the information pattern 30 provided in the abrasion area of the 3D data 103 because the abrasion area of the artificial joint 10 is abraded.

When the 3D data 103 is overlapped with the 2D data 102, the information patterns 30 of the artificial joint 10 of both data coincide with each other. At this time, the 3D data 103 is matched with the 2D data 102 in a matching device such as a PC where a matching program is stored. When the 3D data 103 is matched with the 2D data 102, the 3D data 103 such as a cross-section A-A in FIG. 5B is enlarged, reduced, or rotated at various angles in the matching device by using a zoom function of a mouse or the like to match to the 2D data 102.

The above description is only illustrative of the technical spirit of the present invention, and it will be apparent to those skilled in the art that various modifications, substitutions and alterations can be made hereto without departing from the spirit and scope of the invention as defined by the appended claims. Therefore, the exemplary embodiments of the present invention and the accompanying drawings are provided for illustrative purposes only but not intended to limit the technical concept of the present invention, and the scope of the technical concept of the present invention is not limited thereto according to the exemplary embodiments of the present invention and the accompanying drawings. The protective scope of the present invention should be construed based on the appended claims, and all the technical spirits in the equivalent scope thereof should be construed as falling within the scope of the present invention.

As described above, the exemplary embodiments have been described and illustrated in the drawings and the specification. The exemplary embodiments were chosen and described in order to explain certain principles of the invention and their practical application, to thereby enable others skilled in the art to make and utilize various exemplary embodiments of the present invention, as well as various alternatives and modifications thereof. As it is evident from the foregoing description, certain aspects of the present invention are not limited by the particular details of the examples illustrated herein, and it is therefore contemplated that other modifications and applications, or equivalents thereof, will occur to those skilled in the art. Many changes, modifications, variations and other uses and applications of the present construction will, however, become apparent to those skilled in the art after considering the specification and the accompanying drawings. All such changes, modifications, variations and other uses and applications which do not depart from the spirit and scope of the invention are deemed to be covered by the invention which is limited only by the claims which follow.

## Claims

1. An artificial joint with an abrasion measuring element, wherein abrasion of the artificial joint is determined by any one selected from a sensor device, a marker, and an information pattern provided in the artificial joint.

2. The artificial joint with an abrasion measuring element of claim 1, wherein the sensor devices are provided on a tibial component and a tibial plate configuring the artificial joint.

3. The artificial joint with an abrasion measuring element of claim 2, wherein the sensor devices include
a first sensor provided in a femoral component; and
a second sensor provided in the tibial component,
wherein the abrasion of the artificial joint is determined by distance information between the first sensor and the second sensor acquired from the first sensor and the second sensor.

4. The artificial joint with an abrasion measuring element of claim 3, wherein the first sensor is positioned on one side of the femoral component so as not to interfere with the operation of the artificial joint and the second sensor is positioned directly below the first sensor correspondingly with the first sensor and is positioned on one side of the tibial component so as not to interfere with the operation of the artificial joint.

5. The artificial joint with an abrasion measuring element of claim 3, wherein the first sensor and the second sensor are non-power sensors.

6. The artificial joint with an abrasion measuring element of claim 2, wherein the sensor device is an abrasion amount measuring sensor, and the abrasion amount measuring sensor is positioned on one side of the femoral component or the tibial component so as not to interfere with the operation of the artificial joint to measure an amount of abrasive particles of the tibial plate in a body fluid filled in a glenoid cavity.

7. The artificial joint with an abrasion measuring element of claim 6, wherein the abrasion amount measuring sensor is a non-power sensor.

8. The artificial joint with an abrasion measuring element of claim 3, wherein the first sensor is attached to a lowest point of the femoral component.

9. The artificial joint with an abrasion measuring element of claim 1, wherein the marker is provided at either the femoral component or the tibial plate configuring the artificial joint, or at both the femoral component and the tibial plate.

10. The artificial joint with an abrasion measuring element of claim 9, wherein a plurality of markers or three or more markers are provided.

11. The artificial joint with an abrasion measuring element of claim 9, wherein first artificial joint 3D data is overlapped with 2D data of artificial joint imaging operated to a patient to determine the abrasion of the artificial joint through abrasion area data where the artificial joint of the 2D data is not overlapped with the artificial joint of the 3D data.

12. The artificial joint with an abrasion measuring element of claim 11, wherein artificial joint makers of both data coincide with each other when the 3D data is overlapped with the 2D data.

13. The artificial joint with an abrasion measuring element of claim 11, wherein the 2D data is X-ray or CT data.

14. The artificial joint with an abrasion measuring element of claim 1, wherein any one of the information patterns is provided in a friction region of the artificial joint and the remaining plurality of information patterns is provided in a region where the friction of the artificial joint does not occur, and 3D data of a first artificial joint is overlapped with 2D data of artificial joint imaging operated to a patient to determine the abrasion of the artificial joint through data obtained by comparing the information pattern provided in the friction region of the artificial joint of the 2D data with the information pattern provided in the friction region of the artificial joint of the 3D data.

15. The artificial joint with an abrasion measuring element of claim 14, wherein artificial joint information patterns of both data coincide with each other when the 3D data is overlapped with the 2D data.

16. The artificial joint with an abrasion measuring element of claim 14, wherein product information of the artificial joint is stored in the information pattern.

17. A method for measuring an artificial joint, the method comprising:
acquiring first artificial joint 3D data;
acquiring imaging 2D data of the artificial joint operated to a patient;
overlapping the 3D data with the 2D data; and
determining abrasion of the artificial joint through an abrasion area of the artificial joint not overlapped with each other when the 3D data is overlapped with the 2D data or determining abrasion of the artificial joint through data obtained by comparing the information pattern provided in the friction region of the artificial joint of the 2D data with the information pattern provided in the friction region of the artificial joint of the 3D data when the 3D data is overlapped with the 2D data.

18. The method for measuring an artificial joint of claim 17, wherein artificial joint markers or pattern information of both data coincide with each other when the 3D data is overlapped with the 2D data.
